(19) **Europäisches Patentamt / European Patent Office / Office européen des brevets**

(11) **EP 2 384 701 B1**

(12) **EUROPEAN PATENT SPECIFICATION**

(45) Date of publication and mention
of the grant of the patent:
**19.03.2014  Bulletin 2014/12**

(51) Int Cl.:
*A61B 8/06* (2006.01)     *G01S 15/89* (2006.01)

(21) Application number: **11161807.0**

(22) Date of filing: **11.04.2011**

(54) **Ultrasound imaging device and method for clutter filtering**

Ultraschallbildgebungsvorrichtung und Verfahren zur Störungsfilterung

Dispositif d'imagerie à ultrasons et procédé de filtrage d'écho parasite

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **07.05.2010  KR 20100043154**

(43) Date of publication of application:
**09.11.2011  Bulletin 2011/45**

(73) Proprietor: **Samsung Medison Co., Ltd.
Nam-myun
Hongchun-gun
Kangwon do 250-875 (KR)**

(72) Inventors:
 • **Kim, Tae Yun
   143-824, Seoul (KR)**
 • **Sandstrom, Kurt
   140-200, Seoul (KR)**

(74) Representative: **Schmid, Wolfgang
Lorenz & Kollegen
Patentanwälte Partnerschaftsgesellschaft
Alte Ulmer Strasse 2
89522 Heidenheim (DE)**

(56) References cited:
  **US-A1- 2004 199 078     US-A1- 2006 079 776**

 • **LEDOUX L A F ET AL: "REDUCTION OF THE
   CLUTTER COMPONENT IN DOPPLER
   ULTRASOUND SIGNALS BASED ON SINGULAR
   VALUE DECOMPOSITION: A SIMULATION
   STUDY", ULTRASONIC IMAGING, DYNAMEDIA
   INC., SILVER SPRING, MD, US, vol. 19, no. 1, 1
   January 1997 (1997-01-01), pages 1-18,
   XP000702517, ISSN: 0161-7346**
 • **PEIDONG WANG ET AL: "An Improved Mean
   Frequency Estimator for Ultrasonic Color Flow
   Imaging Using Second-Order Autoregressive
   Model", ENGINEERING IN MEDICINE AND
   BIOLOGY SOCIETY, 2005. IEEE-EMBS 2005. 27T
   H ANNUAL INTERNATIONAL CONFERENCE OF
   THE SHANGHAI, CHINA 01-04 SEPT. 2005,
   PISCATAWAY, NJ, USA,IEEE, 1 September 2005
   (2005-09-01), pages 5643-5646, XP010907173,
   DOI: DOI:10.1109/IEMBS.2005.1615766 ISBN:
   978-0-7803-8741-6**
 • **BASCOM P A J ET AL: "On the doppler signal
   from a steady flow asymmetrical stenosis model:
   Effects of turbulence", ULTRASOUND IN
   MEDICINE AND BIOLOGY, NEW YORK, NY, US,
   vol. 19, no. 3, 1 January 1993 (1993-01-01), pages
   197-210, XP026433912, ISSN: 0301-5629
   [retrieved on 1993-01-01]**
 • **Quinonez: "Real-Time Sensor Anomaly
   Detection", , 20 August 2010 (2010-08-20),
   XP55003838, Retrieved from the Internet: URL:
   http://usrp.usra.edu/technicalPapers/s tennis/
   QuinonezAug10.pdf [retrieved on 2011-07-28]**

EP 2 384 701 B1

**Description**

CROSS-REFERENCE TO RELATED APPLICATION

This application claims the benefit of Korean Patent Application No. 10-2010-0043154, filed on May 7, 2010, in the Korean Intellectual Property Office.

BACKGROUND

1. Field of the Invention

The present invention relates to a Doppler mode ultrasound imaging device and a Doppler mode ultrasonic image processing method.

2. Description of the Related Art

An ultrasound imaging device may irradiate ultrasonic waves onto a human body and measure a Doppler shift frequency of the ultrasonic waves reflected from a bloodstream and thereby, detect a distribution of the bloodstream in real time to display the distribution.

Even when the irradiated ultrasonic waves are focused and concentrated on the bloodstream, a portion of the ultrasonic waves may be propagated in an undesired direction and thus, in addition to a reflected signal from the bloodstream, an undesired signal different from the reflected signal from the bloodstream may return mixed with the reflected signal from the bloodstream. In this instance, generally, the reflected signal from the bloodstream is referred to as a Doppler signal, and the undesired signal from other tissues is referred to as a clutter signal.

In general, when the ultrasonic waves are focused on a focal point, most energy may be transferred to a focal point, and a portion of the ultrasonic waves may leak to the outside of the focal point and thus, a significant portion of return signals may be reflected from the desired focal point. However, since a reflectivity of the bloodstream may be significantly lower than the reflectivity of a neighboring tissue, that is, a vessel wall, a muscle, and the like, a magnitude of the clutter signal may mostly exceed the magnitude of the Doppler signal from the bloodstream even though a minimal amount of ultrasonic waves may leak out.

Accordingly, a number of clutter filtering schemes may effectively eliminate the clutter signal in the existing ultrasonic system when configuring a color Doppler mode.

As the clutter filtering schemes, a scheme of using an infinite impulse response (IIR) type high pass filter in which a cutoff characteristic is predetermined, an adaptive filtering scheme of selecting an optimum cutoff according to a signal characteristic of each pixel, a scheme of eliminating the clutter signal by decomposing a component of ensemble data for each pixel, and the like may be given.

However, in the above schemes, a variance, a mean frequency, a power of a signal before being applied with the clutter filtering, or a variance, a mean frequency, a power of a decomposed signal may mostly be used.

Accordingly, an ultrasound imaging device and method of determining a skewness with respect to an in-phase/quadrature-phase (I/Q) signal in a frequency domain, and configuring a decision logic of a clutter filter using the skewness, is desired.

In US 2004/0199078 Al a system and method for adaptive clutter filtering in ultrasound color flow imaging is provided including an iterative algorithm that is used to select the best clutter filter for each packet of color flow data. If significant clutter motion is present, a high pass filter cutoff frequency is automatically set to suppress the clutter and associated flash artifacts. The cutoff frequency is chosen according to the frequency of the clutter - the lower the clutter frequency, the lower the cutoff frequency can be. If clutter frequencies are low, lower filter cutoffs allow for maximum low flow detection. In this manner, the filter cutoff frequency can be optimized based on the data for each pixel in the color flow image.

Furthermore, reference is made to Ledoux L. A. F. et al : "Reduction of the Clutter Component in Doppler Ultrasound Signals Based on Singular Value Decomposition: A Simulation Study", ULTRASOUND IMAGING, Dynamedia Inc., Silver Spring, MD, US, vol. 19, no. 1, 1 January 1997 (1997-01-01), pages 1-18, XP000702517, ISSN: 0161-7346.

SUMMARY

The invention is defined in the independent claims 1, 6, 9 and 11. Preferred embodiments are defined in the dependent claims.

An aspect of the present invention provides a Doppler mode ultrasound imaging device and a method of computing a skewness of an in-phase/quadrature-phase (I/Q) signal to adaptively perform a clutter filtering according to the computed skewness.

Another aspect of the present invention also provides a Doppler mode ultrasound imaging device and a method of determining whether a Doppler component is dominant or the clutter component is dominant in an I/Q signal based on a skewness of the I/Q signal to perform a clutter filtering.

Still another aspect of the present invention also provides a Doppler mode ultrasound imaging device and a method of selecting a submatrix in which a Doppler component is dominant, among a plurality of submatrices generated by singular value decomposing an I/Q signal, using a skewness of each of the plurality of submatrices, to form a color flow image of an ultrasonic image.

According to an aspect of the present invention, there is provided a. Doppler mode ultrasound imaging device, including a signal converter to emit an ultrasonic signal to an object and receive the ultrasonic signal reflected from the object, and to convert the received ultrasonic signal into an in-phase/quadrature-phase (I/Q) signal corresponding to each pixel of an image in an ultrasonic image, and a control unit to convert the I/Q signal into a frequency domain to compute a skewness, and to apply a clutter filtering to the I/Q signal according to the computed skewness.

According to another aspect of the present invention, there is provided a Doppler mode ultrasound imaging system, including a signal converter to emit an ultrasonic signal to an object and receive the ultrasonic signal reflected from the object, and to convert the received ultrasonic signal into an I/Q signal corresponding to each pixel of an image in an ultrasonic image, and a control unit to perform a singular value decomposition with respect to the I/Q signal to generate a plurality of submatrices, and to form a color flow image of the ultrasonic image using a submatrix in which a Doppler component is dominant, among the plurality of submatrices.

According to still another aspect of the present invention, there is provided a Doppler mode ultrasonic image processing method, including emitting an ultrasonic signal to an object, and converting the ultrasonic signal reflected from the object into an I/Q signal, converting the I/Q signal into a frequency domain to compute a skewness, determining whether a clutter component is dominant in the I/Q signal based on the computed skewness, and applying the clutter filtering to the I/Q signal when the clutter component is determined to be dominant.

According to yet another aspect of the present invention, there is provided a Doppler mode ultrasonic image processing method, including emitting an ultrasonic signal to an object, and converting the ultrasonic signal reflected from the object into an I/Q signal, performing a singular value decomposition with respect to the I/Q signal to generate a plurality of submatrices, converting each of the plurality of submatrices into a frequency domain to compute a skewness of each of the plurality of submatrices, selecting the submatrix in which a Doppler component is dominant, among the plurality of submatrices based on the computed skewness, and forming a color flow image of the ultrasonic image using the selected submatrix in which the Doppler component is dominant.

According to the invention, by filtering a clutter signal according to a skewness of an I/Q signal of an ultrasonic signal reflected from an object, an ultrasonic image may be displayed on a screen using a signal in which a clutter component is filtered, or submatrices in which a Doppler component is dominant.

BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:

FIG. 1 is a block diagram illustrating a configuration of an ultrasonic image device according to an embodiment of the present invention;
FIG. 2 is a block illustrating a configuration of a control unit illustrated in FIG. 1.
FIG. 3 through FIG. 5 are graphs illustrating a skewness used in an ultrasonic image device according to an embodiment of the present invention;
FIG. 6 is a block diagram illustrating a configuration in another example of a control unit illustrated in FIG. 1;
FIG. 7 is a flowchart illustrating a method of processing an ultrasonic image according to an embodiment of the present invention;
FIG. 8 is a flowchart illustrating an operation of performing a clutter filtering of FIG. 7;
FIG. 9 is a flowchart illustrating a method of processing an ultrasonic image according to another embodiment of the present invention; and
FIG. 10 is a flowchart illustrating an operation of selecting submatrices of FIG. 9.

DETAILED DESCRIPTION

Reference will now be made in detail to exemplary embodiments of the present invention, examples of which are illustrated in the accompanying drawings, wherein like reference numerals refer to the like elements throughout.

Exemplary embodiments are described below to explain the present invention by referring to the figures.

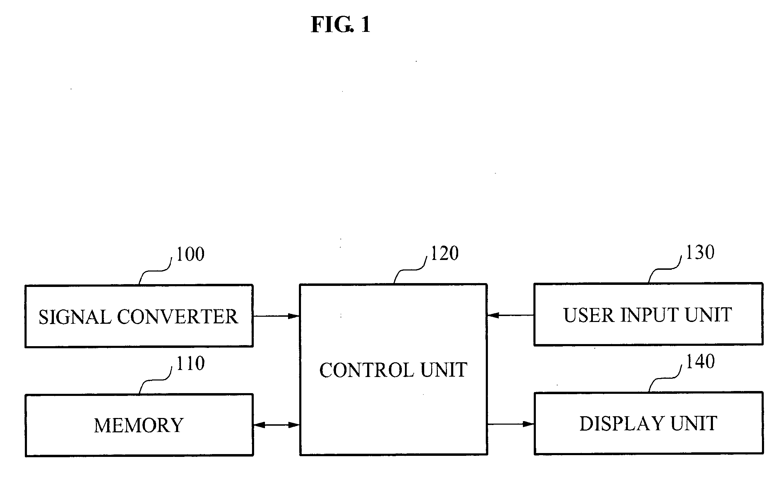

FIG. 1 is a diagram illustrating a configuration of an ultrasonic image device according to an embodiment of the present invention.

Referring to FIG. 1, the ultrasonic image device may include a signal converter 100, a memory 110, a control unit 120, a user input unit 130, and a display unit 140.

The signal converter 100 may emit an ultrasonic signal to an object and receive an ultrasonic signal reflected from the object, that is, an ultrasound echo signal. Then, the signal converter 100 may convert the received ultrasonic signal into an in-phase/quadrature-phase (I/Q) signal corresponding to each pixel of an image in an ultrasonic image and may output the I/Q signal. In this instance, in addition to a Doppler component, a clutter component may be contained in the I/Q signal.

The signal converter 100 may sequentially and repeatedly perform a process of forming a transmission signal based on an ensemble number, thereby generating a plurality of transmission signals. The signal converter 100 may convert the generated transmission signal into an ultrasonic signal and may transmit the converted ultrasonic signal to the object. Then, when ultrasound echo signal reflected from the object is received, the signal converter 100 may convert the ultrasound echo signal into a digital signal, and may convert the converted ultrasound echo signal into an I/Q signal corresponding to each pixel of the image in the ultrasonic image.

The memory 110 may store signals while performing procedures of an ultrasound imaging system. The memory 110 may be implemented as at least one of a general hard disk, random access memory (RAM), and read-only memory (ROM).

The control unit 120 may convert the I/Q signal converted and outputted by the signal converter 100 into a frequency domain to compute a skewness, and may apply a clutter filtering to the I/Q signal according to the computed skewness.

In this instance, the control unit 120 may filter the I/Q signal using a conventional clutter filter. In this case, the memory 110 may store a procedure of a plurality of clutter filterings, and may further store an index and cutoff corresponding to each of the plurality of clutter filterings.

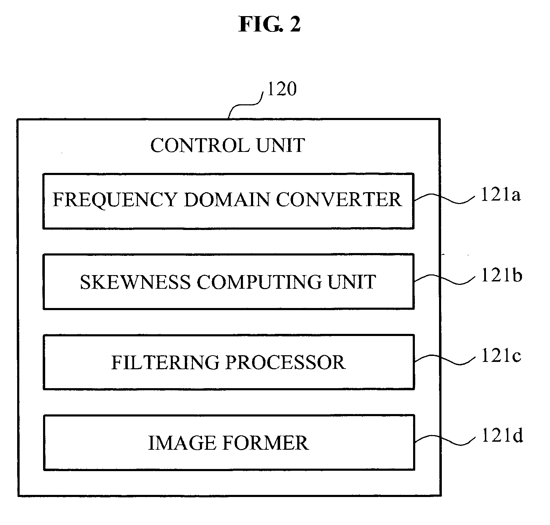

As shown in FIG. 2, the control unit 120 may include a frequency domain converter 121a, a skewness computing unit 121b, a filtering processor 121c, and an image former 121d.

The frequency domain converter 121a may perform a fast Fourier transform (FFT) with respect to the I/Q signal outputted from the signal converter 100 to convert the fast Fourier transformed I/Q signal into a frequency domain. In particular, the frequency domain converter 121a may perform the FFT with respect to the I/Q signal associated with a predetermined ensemble data in the I/Q signal, and may output the I/Q signal in the frequency domain.

The skewness computing unit 121b may compute a skewness based on the I/Q signal converted into the frequency domain. In this instance, the skewness may be computed using at least one of a mean frequency, a variance, a normalized pulse recurrence frequency (Norm. PRF), and an FFT order of the I/Q signal calculated using an autocorrelation of the I/Q signal.

In particular, the skewness may be computed by the following Equation 1.

[Equation 1]

$$\text{Skewness} = \frac{\frac{\text{FFT order}}{2}}{\left(\frac{\text{FFT order}}{2} - 1\right)\left(\frac{\text{FFT order}}{2} - 2\right)} \times \sum_{i=0}^{\frac{\text{FFT order}}{2} - 1} \left(\frac{(x[i]) - \mu}{\sigma}\right)^3$$

FFT order FFT order Here, i may be defined as $i = [0, 1, \ldots, \frac{\text{FFT order}}{2} - 2, \frac{\text{FFT order}}{2} - 1]$ and,

x[i] may be defined as $x[i] = \frac{1}{\text{FFT order}} \times i$ FFT order indicates the fast Fourier transform order, Norm. PRF indicates the normalized pulse recurrence frequency, i indicates an index value of x buffer data, and x indicates a value of an x-axis (frequency domain) of a discrete Fourier transform (DFT). $\mu$ indicates the mean frequency, and $\sigma$ indicates

a standard deviation $(\sqrt{\text{variance}})$.

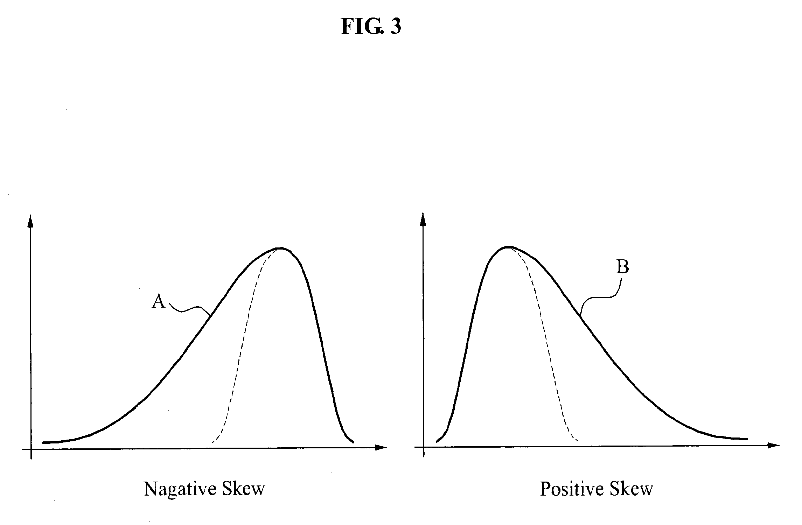

The skewness may be described through graphs illustrated in FIG. 3 through FIG. 5.

Referring to FIG. 3, I/Q signals A and B converted into a frequency domain are illustrated.

In a case of signal A, a curve may lean to the right side since the Doppler component is dominant. In this case, the skewness is computed as a negative skew. In a case of signal B, the curve may lean to the left side since the clutter component is dominant. In this case, the skewness is computed as a positive skew.

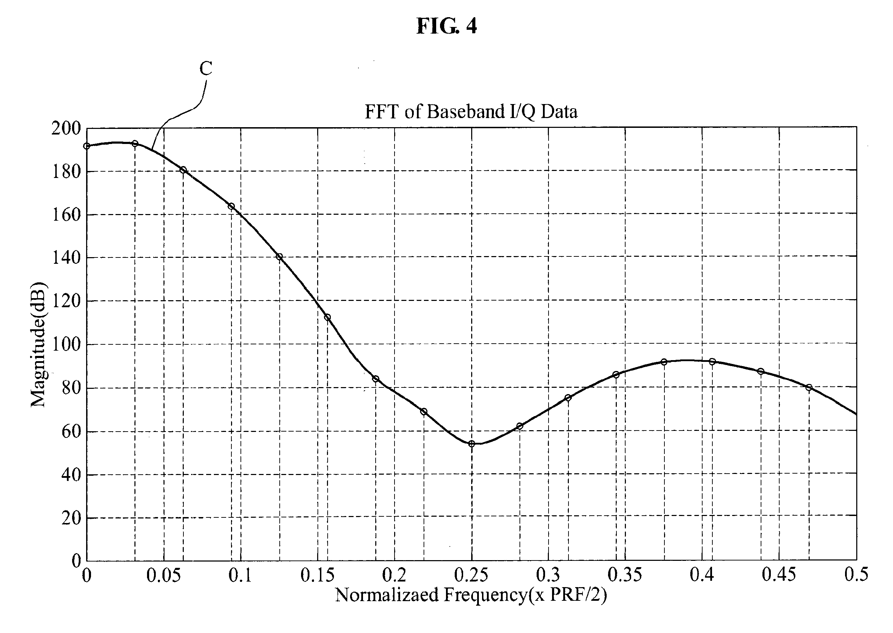

Referring to FIG. 4, curve C is illustrated in a frequency domain of the I/Q signal corresponding to signal A. In this case, the skewness computing unit 121b may compute the mean frequency as 0.0177, and the variance as 0.3968, using the autocorrelation. The skewness computing unit 121b may compute the skewness as 0.4158 using the computed mean frequency and the variance, and Equation 1.

In this instance, since the computed skewness 0.4158 corresponds to a positive value, the clutter component may be determined to be dominant in the I/Q signal, and curve C may be determined to lean to the right side.

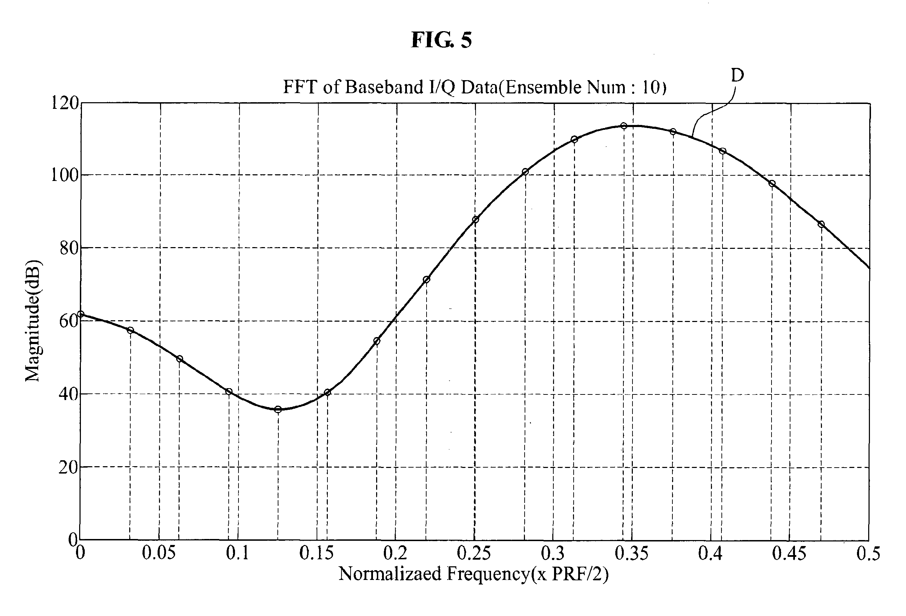

Referring to FIG. 5, curve D is illustrated in the frequency domain of the I/Q signal corresponding to signal B. In this case, the skewness computing unit 121b may compute the mean frequency as 0.3364, and the variance as 0.4730, using the autocorrelation. The skewness computing unit 121b may compute the skewness as -0.0278 using the computed mean frequency and the variance, and Equation 1.

In this instance, since the computed skewness, that is -0.0278, is a negative value, the Doppler component may be determined to be dominant in the I/Q signal, and curve D may be determined to lean to the left side.

The filtering processor 121c may determine whether the clutter component is dominant in the I/Q signal based on the skewness computed by the skewness computing unit 121b, and may apply the clutter filtering when the clutter component is determined to be dominant.

In this instance, the filtering processor 121c may use the computed skewness to recognize that the clutter component is dominant in the I/Q signal when the skewness is a positive number or equal to or greater than a predetermined threshold value. In contrast, when the skewness is a negative number or equal to or less than the predetermined threshold value, the filtering processor 121c may recognize that the clutter component is not dominant and the Doppler component is dominant.

When the filtering processor 121c determines that the clutter component is dominant in the I/Q signal, the filtering processor 121c may apply, to the I/Q signal, the clutter filtering of a predetermined index or a predetermined cutoff.

Thereafter, the frequency domain converter 121a may perform a FFT of(*"transform" / "fast Fourier transform"? *) the filtered I/Q signal into the frequency domain, and the skewness computing unit 121b may compute the skewness again based on data in which the filtered I/Q signal is fast Fourier transformed into the frequency domain. The filtering processor 121c may determine whether the clutter component is dominant in the transformed data based on the recomputed (*again computed skewness, and may apply the clutter filtering of the subsequent index to the I/Q signal when the clutter component is dominant.

When the clutter component is determined not to be dominant in the I/Q signal, the filtering processor 121 c may apply, to the filtered I/Q signal or to the unfiltered I/Q signal, the clutter filtering corresponding to the lowest cutoff among a plurality of cutter filterings stored in the memory 110.

The filtering processor 121c may determine whether the cutter component is dominant in the I/Q signal based on the skewness, and may read out the clutter filtering having a higher cutoff as the clutter component becomes more dominant, and apply the read clutter filtering to the I/Q signal.

The image former 121d may form a color flow image of an ultrasonic image using the I/Q signal finally filtered by the filtering processor 121c, and may transfer the formed color flow image to the display unit 140.

The control unit 120 may filter the clutter component in the I/Q signal using a Hankel singular value decomposition (Hankel SVD).

In this case, the control unit 120 may perform a singular value decomposition with respect to the I/Q signal converted by the signal converter 100 of FIG. 1, and may form the color flow image of the ultrasonic image using submatrices in which the Doppler component is dominant, among the plurality of submatrices.

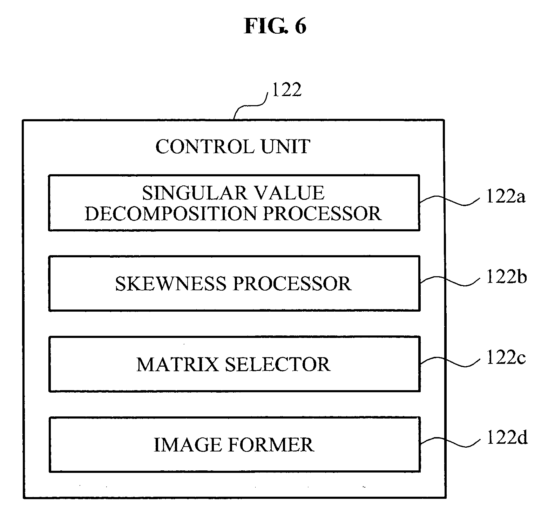

As illustrated in FIG. 6, the control unit 120 may include a singular value decomposition processor 122a, a skewness processor 122b, a matrix selector 122c, and an image former 122d.

The singular value decomposition processor 122a may perform a singular value decomposition with respect to the I/Q signal converted by the signal converter 100 to generate a plurality of submatrices.

The skewness processor 122b may perform an FFT of each of the plurality of submatrices, and may compute the skewness corresponding to each of the plurality of submatrices using each of converted data.

The matrix selector 122c may determine whether the Doppler component is dominant in each of the plurality of submatrices, based on the skewness computed by the skewness processor 122b, and may select submatrices in which the Doppler component is determined to be dominant.

In this instance, the matrix selector 122c may recognize that the Doppler component is dominant in a case where the skewness is a negative number or less than a predetermined threshold value.

The image former 122d may form a color flow image of an ultrasonic image using a submatrix selected by the matrix selector 122c, and may transfer the formed color flow image to the display unit 140.

As a result, since the submatrices in which the clutter component is dominant are not selected by the matrix selector 122c, the color flow image of the ultrasonic image may be formed by submatrices in which the clutter component is not dominant, that is, submatrices in which the Doppler component is dominant.

The user input unit 130 may provide an interface receiving input information of a user. In the present exemplary embodiment, the interface may enable the user to select a size and location information of a region of interest, that is, a color box set in a B mode (brightness mode) region of the object. The user input unit 130 may include a control panel, a mouse, a keyboard, and the like.

The display unit 140 may display the color flow image formed by the control unit 120 on a screen for the user.

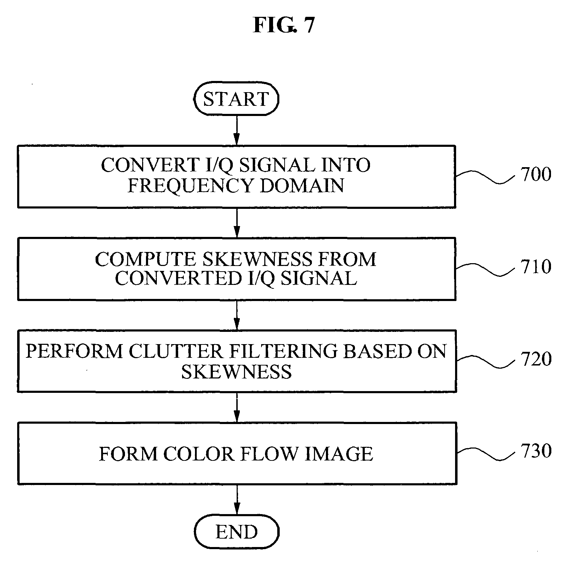

FIG. 7 is a flowchart illustrating a method of processing an ultrasonic image according to an embodiment of the present invention.

Referring to FIG. 7, in operation 700, an ultrasonic signal may be emitted to an object, and the ultrasonic signal reflected from the object may be converted into an I/Q signal. In operation 700, an FFT is performed with respect to the I/Q signal associated with a predetermined ensemble data in the converted I/Q signal to transform the converted I/Q signal into an I/Q signal in a frequency domain.

In operation 710, a skewness is computed from the I/Q signal transformed in operation 700. In operation 710, a mean frequency, a variance, a normalized pulse recurrence frequency (Norm. PRF), and an FFT order of the I/Q signal may be calculated from the converted I/Q signal, and the skewness may be computed using each of the calculated values.

In operation 720, the clutter filtering may be performed with respect to the I/Q signal according to whether the clutter component is dominant in the I/Q signal based on the skewness computed in operation 710.

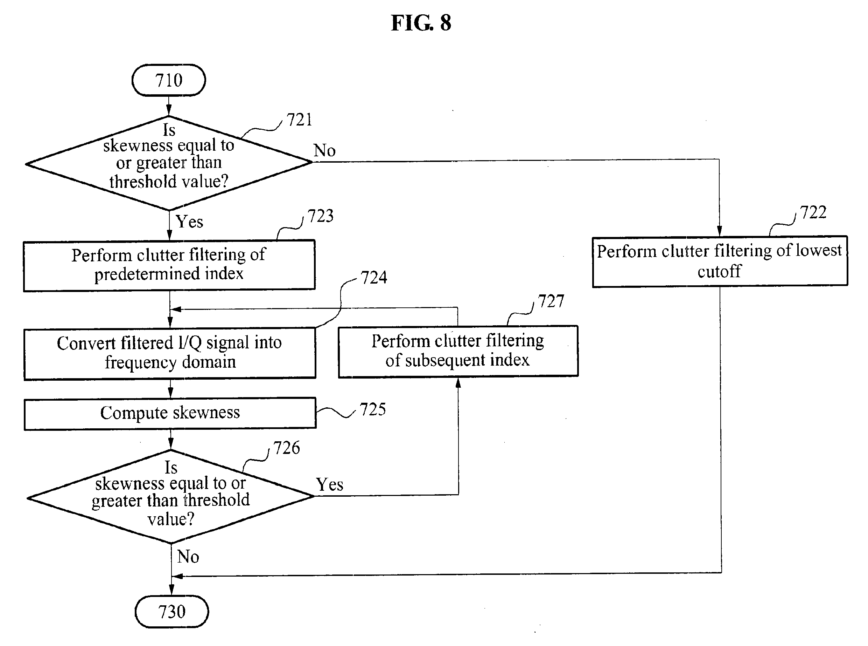

In this instance, operation 720 may include several operations illustrated in FIG. 8.

FIG. 8 corresponds to a case where a device, performing a method of processing an ultrasonic image according to an embodiment of the present invention, stores, in the memory 110, a plurality of clutter filterings and indexes and cutoff values of the plurality of clutter filterings. The device may be implemented by recognizing whether the clutter component is dominant based on whether the skewness of the I/Q signal is equal to or greater than a predetermined threshold value.

Referring to FIG. 8, whether the skewness computed in operation 710 is equal to or greater than the threshold value may be determined in operation 721.

When the skewness is determined to be equal to or greater than the threshold value in operation 721, a clutter filtering having the lowest cutoff value, among the plurality of clutter filterings, may be performed with respect to the I/Q signal.

In contrast, when the skewness is determined to be equal to or less than the threshold value in operation 721, a clutter filtering having a predetermined index, among the plurality of clutter filterings, may be performed with respect to the I/Q signal.

In operation 724, the I/Q signal filtered in operation 723 may be transformed in a frequency domain through a FFT.

In operation 725, the skewness may be computed using the I/Q signal transformed in operation 724.

In operation 726, whether the skewness computed in operation 725 is equal to or greater than the threshold value may be determined. As a result of the determination, in a case where the skewness is equal to or greater than the threshold value, the clutter filtering of the subsequent index may be performed with respect to the filtered I/Q signal in operation 727.

As a result, the clutter filtering may be repeatedly performed in operation 721 through operation 725, until the skewness of the I/Q signal transformed in operation 700 becomes equal to or less than the threshold value.

In operation 730, a color flow image of an ultrasonic image may be formed using the I/Q signal finally filtered in operation 720, and the formed color flow image may be displayed.

The color flow image displayed in operation 730 may be formed by signals in which the clutter filtering is repeatedly performed until the skewness of the I/Q signal becomes equal to or greater than the threshold value and thereby provides a more accurate ultrasonic image to a user.

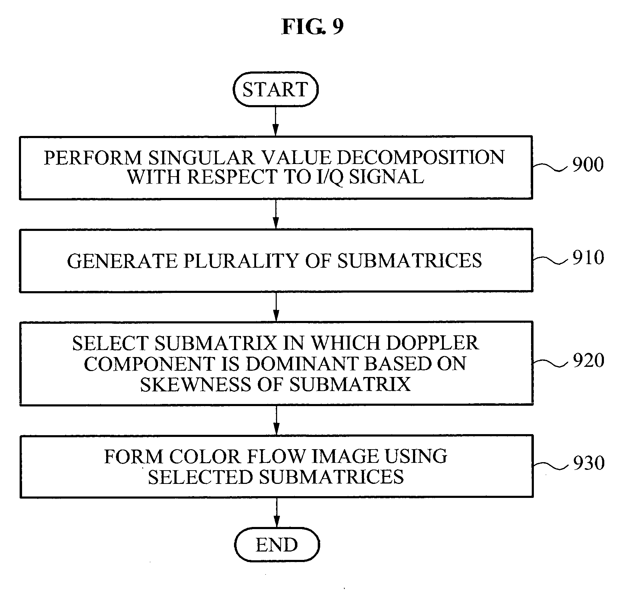

FIG. 9 is a flowchart illustrating a method of processing an ultrasonic image according to another embodiment of the present invention. In this case, the method of processing the ultrasonic image may filter a clutter component in the I/Q signal using a Hankel singular value decomposition (Hankel SVD).

Referring to FIG. 9, in operation 900, an ultrasonic signal may be emitted to an object, and the ultrasonic signal reflected from the object may be converted into an I/Q signal. In operation 900, the converted I/Q signal may be singular value decomposed.

In operation 910, a plurality of submatrices may be generated from the singular value decomposed I/Q signals.

In operation 920, submatrices in which a Doppler component is dominant may be selected based on a skewness of each of the plurality of submatrices generated in operation 910.

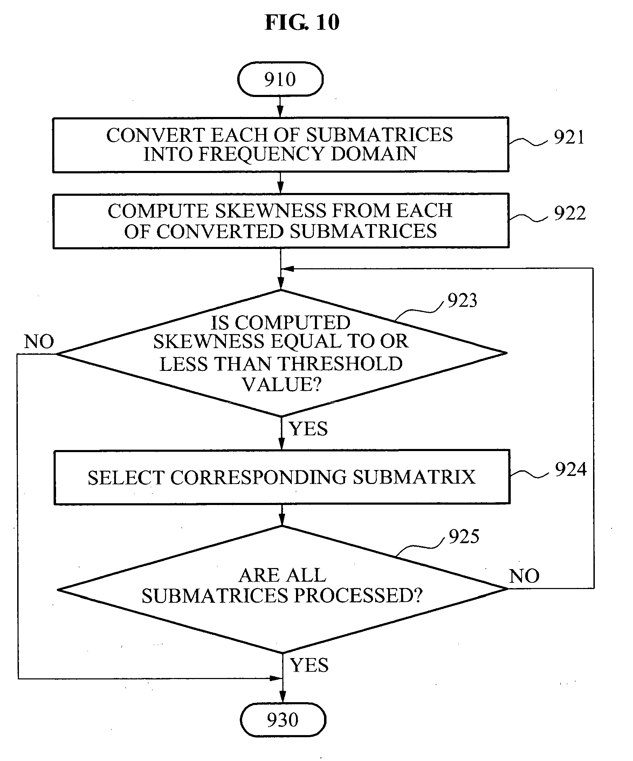

Operation 920 may include several operations described with reference to FIG. 10.

Referring to FIG. 10, in operation 921, each of the plurality of submatrices may be transformed into a frequency domain through a FFT.

In operation 922, a skewness may be computed from each of the plurality of submatrices transformed in operation 921.

In operation 923, whether the skewness computed in operation 922 is equal to or less than a threshold value may be determined.

As a determination result of operation 923, in a case where the skewness is determined to be equal to or less than the threshold value, a submatrix having the skewness equal to or less than the threshold value may be considered as a submatrix in which the Doppler component is dominant and select the submatrix in operation 924.

In operation 925, whether the plurality of submatrices are all processed in operation 923 may be determined. When not all of the plurality of submatrices are processed, whether the skewness of the subsequent matrix is equal to or less than the threshold value may be determined in operation 923.

In a case where operation 922 does not compute each skewness of the plurality of submatrices at one time, and each skewness is individually computed, the skewness of the subsequent submatrix may be computed in operation 922 when not all of the plurality of submatrices are determined to be processed in operation 925.

As a result, submatrices in which the Doppler component is dominant, among the plurality of submatrices, may be sorted and selected, using the skewness of the plurality of submatrices, in operation 921 through operation 925.

In operation 930, a color flow image of an ultrasonic image may be formed using the submatrices selected in operation 920, and the formed color flow image may be displayed.

The color flow image displayed in operation 930 may be formed by submatrices in which the Doppler component is dominant, that is, the clutter component is not dominant, among the plurality of submatrices of the I/Q signal and thus, more accurate ultrasonic image may be provided to a user.

The above-described exemplary embodiments of the present invention may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described exemplary embodiments of the present invention, or vice versa.

Although a few exemplary embodiments of the present invention have been shown and described, the present invention is not limited to the described exemplary embodiments. Instead, it would be appreciated by those skilled in the art that changes may be made to these exemplary embodiments without departing from the principles of the invention, the scope of which is defined by the claims and their equivalents.

**Claims**

1. A Doppler mode ultrasound imaging device, comprising:

   a signal converter (100) adapted to emit an ultrasonic signal to an object and to receive the ultrasonic signal reflected from the object, and configured to convert the received ultrasonic signal into an in-phase/quadrature-phase (I/Q) signal corresponding to each pixel of an image in an ultrasonic image; and
   a control unit (120) adapted to convert the I/Q signal into a frequency domain **characterised in that** the control unit is configured to compute a skewness, and to apply a clutter filtering to the I/Q signal according to the computed skewness.

2. The ultrasound imaging device of claim 1, wherein the control unit (120) is adapted to determine whether a clutter component is dominant in the I/Q signal based on the skewness, and to apply the clutter filtering when the clutter component is determined to be dominant.

3. The ultrasound imaging device of claim 1, wherein the skewness is computed using at least one of a mean frequency, a variance, a normalized pulse recurrence frequency, and a fast Fourier transform (FFT) order of the I/Q signal.

4. The ultrasound imaging device of claim 1, further comprising
a memory (110) adapted to store a plurality of clutter filterings and cutoffs of each of the plurality of clutter filterings, wherein
the control unit (120) is adapted to determine a degree of dominance of a clutter component in the I/Q signal based on the skewness, and to apply, to the I/Q signal, a clutter filtering having a higher cutoff as the clutter component becomes more dominant.

5. The ultrasound imaging device of claim 1, further comprising:

a memory (110) adapted to store a plurality of clutter filterings and sequential indexes of each of the plurality of clutter filterings, wherein
the control unit (120) is adapted to apply, to a filtered I/Q signal, a clutter filtering corresponding to a subsequent sequential index among the plurality of clutter filterings according to the skewness of the filtered I/Q signal, when the I/Q signal is filtered by one of the plurality of clutter filterings.

6. A Doppler mode ultrasound imaging system, comprising:

a signal converter (100) adapted to emit an ultrasonic signal to an object and to receive the ultrasonic signal reflected from the object, and to convert the received ultrasonic signal into an in-phase/quadrature-phase (I/Q) signal corresponding to each pixel of an image in an ultrasonic image; and
a control unit (120) adapted to perform a singular value decomposition with respect to the I/Q signal to generate a plurality of submatrices, **characterised in that** the control unit is configured to form a color flow image of the ultrasonic image using a submatrix in which a Doppler component is dominant, being selected based on a computed skewness among the plurality of submatrices.

7. The ultrasound imaging system of claim 6, wherein the control unit (120) is adapted to convert each of the plurality of submatrices into a frequency domain to compute a skewness of each of the plurality of submatrices, and to select the submatrix in which the Doppler component is dominant, among each of the plurality of submatrices based on the computed skewness.

8. The ultrasound imaging system of claim 6, wherein the skewness is computed using at least one of a mean frequency, a variance, a normalized pulse recurrence frequency, and a fast Fourier transform (FFT) order of the I/Q signal.

9. A Doppler mode ultrasonic image processing method, comprising the steps:

emitting an ultrasonic signal to an object, and converting the ultrasonic signal reflected from the object into an in-phase/quadrature-phase (I/Q) signal;
converting the I/Q signal into a frequency domain;
determining whether a clutter component is dominant in the I/Q signal; and
applying the clutter filtering to the I/Q signal when the clutter component is determined to be dominant, **characterised in that** the skewness of the I/Q signal into the frequency domain is computed and the determining whether a clutter component is dominant in the I/Q signal is based on the computed skewness.

10. The method of claim 9, wherein the skewness has a value indicating whether a Doppler component is dominant or the clutter component is dominant, based on a curved shape of the frequency domain.

11. A Doppler mode ultrasonic image processing method, **characterized by**:

emitting an ultrasonic signal to an object, and converting the ultrasonic signal reflected from the object into an in-phase/quadrature-phase (I/Q) signal;
performing a singular value decomposition with respect to the I/Q signal to generate a plurality of submatrices;
converting each of the plurality of submatrices into a frequency domain
selecting the submatrix in which a Doppler component is dominant, among the plurality of submatrices and forming a color flow image of the ultrasonic image using the selected submatrix in which the Doppler component is dominant,
**characterised in that** the skewness of each of the plurality of submatrices is computed and the selecting the submatrix in which a Doppler component is dominant, among the plurality of submatrices is based on the computed skewness.

**EP 2 384 701 B1**

**Patentansprüche**

1. Doppler-Modus-Ultraschalfbildgebungsvorrichtung, welche Folgendes aufweist:

   einen Signal-Konverter (100), der dafür vorgesehen ist, ein Ultraschallsignal zu einem Objekt auszusenden und das von dem Objekt reflektierte Ultraschallsignal zu empfangen, und der dafür vorgesehen ist, das empfangene Ultraschallsignal in ein In-Phasen/Quadratur-Phasen-(I/Q)-Signal zu konvertieren, das jedem Pixel einer Abbildung in einem Ultraschallsignal entspricht; und
   eine Steuereinheit (120), die dafür vorgesehen ist, das I/Q-Signal in einen Frequenzbereich zu konvertieren,
   **dadurch gekennzeichnet, dass**
   die Steuereinheit dafür vorgesehen ist, eine Asymmetrie zu berechnen und eine Clutterfilterung bzw. Störfilterung gemäß der berechneten Asymmetrie an dem I/Q-Signal anzuwenden.

2. Ultraschallbildgebungsvorrichtung nach Anspruch 1, wobei die Steuereinheit (120) dafür vorgesehen ist, basierend auf der Asymmetrie zu ermitteln, ob eine Störkomponente in dem I/Q-Signal dominant ist, und die Störfilterung anzuwenden, wenn ermittelt wurde, dass die Störkomponente dominant ist.

3. Ultraschallbildgebungsvorrichtung nach Anspruch 1, wobei die Asymmetrie unter Verwendung von wenigstens einer von einer Mittelfrequenz, einer Abweichung, einer normalisierten Pulswiederholfrequenz und einer schnallen Fourier-Transformationsfolge (FFT) des I/Q-Signals berechnet wird.

4. Ultraschallbildgebungsvorrichtung nach Anspruch 1, welche des Weiteren Folgendes aufweist:

   einen Speicher (110), der dafür vorgesehen ist, eine Vielzahl von Störfilterungen und Abschirmungen von jeder der Vielzahl von Störfilterungen zu speichern, wobei
   die Steuereinheit (120) dafür vorgesehen ist, einen Grad der Dominanz einer Störkomponente in dem I/Q-Signal basierend auf der Asymmetrie zu ermitteln,
   und an dem I/Q-Signal eine Störfilterung anzuwenden, die eine höhere Abschirmung aufweist, wenn die Störkomponente dominanter wird.

5. Ultraschallbildgebungsvorrichtung nach Anspruch 1, welche des Weiteren Folgendes aufweist:

   einen Speicher (110), der dafür vorgesehen ist, eine Vielzahl von Störfilterungen und sequenzielle Indizes von jeder der Vielzahl von Störfilterungen zu speichern, wobei
   die Steuereinheit (120) dafür vorgesehen ist, an einem gefilterten I/Q-Signal eine Störfilterung anzuwenden, die einem nachfolgenden sequenziellen Index unter der Vielzahl von Störfilterungen gemäß der Asymmetrie des gefilterten I/Q-Signals entspricht, wenn das I/Q-Signal von einer der Vielzahl von Störfilterungen gefiltert wird.

6. Doppler-Modus-Ultraschallbildgebungssystem, welches Folgendes aufweist:

   einen Signalkonverter (100), der dafür vorgesehen ist, ein Ultraschallsignal zu einem Objekt auszusenden und das von dem Objekt reflektierte Ultraschallsignal zu empfangen, und das empfangene Ultraschallsignal in ein In-Phasen/Quadratur-Phasen-(I/Q)-Signal zu konvertieren, das jedem Pixel einer Abbildung in einem Ultraschallsignal entspricht; und
   eine Steuereinheit (120), die dafür vorgesehen ist, eine Singulärwertzerlegung unter Bezugnahme auf das I/Q-Signal durchzuführen, um eine Vielzahl von Submatrixen zu erzeugen,
   **dadurch gekennzeichnet, dass**
   die Steuereinheit dafür vorgesehen ist, ein Farbströmungsbild des Ultraschallbilds unter Verwendung einer Submatrix zu erzeugen, in welcher eine Doppler-Komponente dominant ist, die, basierend auf einer berechneten Asymmetrie, unter der Vielzahl von Submatrixen ausgewählt ist.

7. Ultraschallbildgebungssystem nach Anspruch 6, wobei die Steuereinheit (120) dafür vorgesehen ist, jede der Vielzahl von Submatrixen in einen Frequenzbereich zu konvertieren, um eine Asymmetrie von jeder der Vielzahl von Submatrixen zu berechnen, und die Submatrix, in welcher die Doppler-Komponente dominant ist, unter jeder der Vielzahl von Submatrixen basierend auf der berechneten Asymmetrie auszuwählen.

8. Ultraschallbildgebungssystem nach Anspruch 6, wobei die Asymmetrie unter Verwendung von wenigstens einer von einer Mittelfrequenz, einer Abweichung, einer normalisierten Pulswiederholfrequenz und einer schnellen Fourier-Transformationsfolge (FFT) des I/Q-Signals berechnet wird.

9. Doppler-Modus-Ultraschallbildgebungsverfahren, welches folgende Schritte aufweist:

Aussenden eines Ultraschallsignals zu einem Objekt und Konvertieren des von dem Objekt reflektierten Uitraschalisignals in ein In-Phasen/Quadratur-Phasen-(I/Q)-Signal;
Konvertieren des I/Q-Signals in einen Frequenzbereich;
Ermitteln, ob eine Störkomponente in dem I/Q-Signal dominant ist; und
Anwenden der Clutterfilterung bzw. Störfilterung an dem I/Q-Signal, wenn ermittelt wird, dass die Störkomponente dominant ist,
**dadurch gekennzeichnet, dass**
die Asymmetrie des I/Q-Signals in den Frequenzbereich berechnet wird, und das Ermitteln, ob eine Störkomponente in dem I/Q-Signal dominant ist, auf der berechneten Asymmetrie basiert.

10. Verfahren nach Anspruch 9, wobei die Asymmetrie einen Wert hat, der anzeigt, ob die Doppler-Komponente dominant ist oder ob die Störkomponente dominant ist, und zwar basierend auf einer gekrümmten Form des Frequenzbereichs.

11. Doppler-Modus-Ultraschallbildgebungsverfahren, **gekennzeichnet durch**:

Aussenden eines Ultraschallsignals zu einem Objekt und Konvertieren des von dem Objekt reflektierten Ultraschallsignals in ein In-Phasen/Quadratur-Phasen-(I/Q)-Signal;
Durchführen einer Singulärwertzertegung unter Bezugnahme auf das I/Q-Signal, um eine Vielzahl von Submatrixen zu erzeugen;
Konvertieren von jeder der Vielzahl von Submatrixen in einen Frequenzbereich; Auswählen der Submatrix, in welcher eine Doppler-Komponente dominant ist, unter der Vielzahl von Submatrixen; und
Erzeugen eines Farbströmungsbilds des Ultraschallbilds unter Verwendung der ausgewählten Submatrix, in welcher die Doppler-Komponente dominant ist,
**dadurch gekennzeichnet**, dass
die Asymmetrie von jeder der Vielzahl von Submatrixen berechnet wird und das Auswählen der Submatrix, in welcher eine Doppler-Komponente dominant ist, unter der Vielzahl von Submatrixen auf der berechneten Asymmetrie basiert.

**Revendications**

1. Dispositif de création d'une image ultrasonique Doppler comportant :

un convertisseur (100) de signal agencé pour émettre un signal ultrasonique vers un objet et pour recevoir un signal réfléchi de l'objet, et configuré pour recevoir un signal ultrasonique dans un signal en-phase/quadrature de phase (I/Q) correspondant à chaque pixel image d'une image ultrasonique ; et
une unité de contrôle (120) agencée pour convertir le signal I/Q en un domaine de fréquence,
**caractérisé en ce que** l'unité de contrôle est configurée pour calculer une asymétrie et pour appliquer un filtrage de défauts du signal I/Q correspondant à l'asymétrie calculée.

2. Dispositif de création d'une image ultrasonique selon la revendication 1, dans lequel l'unité de contrôle (120) est configurée pour déterminer si un composant de défaut est dominant dans le signal I/Q basé sur l'asymétrie et pour appliquer le filtrage de défaut lorsque le composant de défaut est déterminé comme dominant.

3. Dispositif de création d'une image ultrasonique selon la revendication 1, dans lequel l'asymétrie est calculée en utilisant au moins une fréquence majeure, une variante, une fréquence d'impulsion récurrente et un ordre de transformée rapide de Fourrier (FFT) du signal I/Q.

4. Dispositif de création d'une image ultrasonique selon la revendication 1, comportant en outre :

une mémoire (110) agencée pour mémoriser une pluralité de filtrages de défauts et de coupures de chacune des pluralités de filtrages de défauts, dans lequel,
l'unité de contrôle (120) est agencée pour déterminer un degré de dominance de composant de défaut dans le signal I/Q basé sur l'asymétrie et pour appliquer au signal I/Q un filtrage de défaut ayant une coupure plus élevée lorsque le composant de défaut devient moins dominant.

5. Dispositif de création d'une image ultrasonique selon la revendication 1, comportant en outre:

une mémoire (110) agencée pour mémoriser une pluralité de filtrages de défauts et pour indexer séquentiellement chacun des filtrages de défauts de ladite pluralité de filtrages de défauts, dans lequel l'unité de contrôle (120) est agencée pour appliquer à un signal I/Q filtré un filtrage de défaut correspondant à un indice séquentiel subséquent de la pluralité de filtrages de défauts selon l'asymétrie du signal I/Q, lorsque le signal I/Q est filtré par un de la pluralité de filtrages de défauts.

6. Système d'imagerie ultrasonique en mode Doppler comportant :

un convertisseur (100) de signal agencé pour émettre un signal ultrasonique vers un objet et pour recevoir un signal réfléchi de l'objet, et configuré pour convertir le signal ultrasonique reçu en un signal en-phase/quadrature de phase (I/Q) correspondant à chaque pixel image d'une image ultrasonique ; et une unité de contrôle (120) agencée pour effectuer une décomposition de valeur singulière par rapport au signal I/Q pour générer une pluralité de sous-matrices, **caractérisé en ce que** l'unité de contrôle est configurée pour former un flux d'images de couleurs de l'image ultrasonique utilisant une sous-matrice dans laquelle un composant Doppler est dominant, étant sélectionné sur une asymétrie calculée parmi la pluralité de sous-matrices.

7. Système d'imagerie ultrasonique selon la revendication 6, dans lequel l'unité de contrôle (120) est agencée pour convertir chacune des pluralités de sous-matrices en un domaine de fréquences pour calculer une asymétrie de chacune de la pluralité de sous-matrices et pour sélectionner la sous-matrice dans laquelle le composant Doppler est dominant parmi chacune des pluralités de sous-matrices, sur la base de l'asymétrie calculée.

8. Système d'imagerie ultrasonique selon la revendication 6, dans lequel l'asymétrie est calculée en utilisant au moins une des fréquences principales, une variance, une fréquence d'impulsion de récurrence normalisée et un ordre de transformée de Fourrier rapide (FFT) du signal I/Q.

9. Procédé de traitement d'image ultrasonique en mode Doppler, comportant les étapes consistant à :

émettre un signal ultrasonique vers un objet et recevoir un signal réfléchi de l'objet, sous forme d'un signal en-phase/quadrature de phase (I/Q); convertir le signal I/Q en un domaine de fréquence, déterminer si le défaut est dominant dans le signal I/Q, et appliquer le filtrage de défaut au signal I/Q lorsque le défaut est déterminé comme étant dominant, **caractérisé en ce que** l'asymétrie du signal I/Q dans le domaine de fréquence est calculé et il est déterminé si le composant de défaut est dominant dans le signal I/Q sur la base de l'asymétrie calculée.

10. Procédé selon la revendication 9, dans lequel l'asymétrie a une valeur qui indique soit si un composant Doppler est dominant, soit si un composant de défaut est dominant, sur la base d'une courbe du domaine de fréquence.

11. Procédé de traitement d'image ultrasonique en mode Doppler, **caractérisé par** :

l'émission d'un signal ultrasonique vers un objet et la conversion du signal ultrasonique réfléchi par l'objet en un signal en phase/en quadrature de phase (I/Q) ; la décomposition en valeur singulière par rapport au signal (I/Q) pour générer une pluralité de sous-matrices ; la conversion de chacune de la pluralité de sous-matrices en un domaine de fréquences ; la sélection de sous-matrices dans laquelle un composant Doppler parmi la pluralité de sous-matrices, est dominant, et la formation d'un flux d'images couleurs de l'image ultrasonique utilisant la sous-matrice sélectionnée dans laquelle le composant Doppler est dominant ;

**caractérisé en ce que** l'asymétrie de chacune des pluralités de sous-matrices est calculée et la sous-matrice dans laquelle le composant Doppler est dominant, est sélectionnée parmi la pluralité de sous-natices sur la base de l'asymétrie calculée.

**FIG. 1**

EP 2 384 701 B1

# FIG. 2

120

CONTROL UNIT

FREQUENCY DOMAIN CONVERTER — 121a

SKEWNESS COMPUTING UNIT — 121b

FILTERING PROCESSOR — 121c

IMAGE FORMER — 121d

**FIG. 3**

FIG. 4

FFT of Baseband I/Q Data

## FIG. 5

FFT of Baseband I/Q Data(Ensemble Num : 10)

EP 2 384 701 B1

**FIG. 6**

```
                    122

┌──────────────────────────────────────┐
│            CONTROL UNIT                │
│  ┌────────────────────────────────┐   │
│  │       SINGULAR VALUE            │──┐── 122a
│  │  DECOMPOSITION PROCESSOR        │  │
│  └────────────────────────────────┘   │
│  ┌────────────────────────────────┐   │
│  │      SKEWNESS PROCESSOR         │──┐── 122b
│  └────────────────────────────────┘   │
│  ┌────────────────────────────────┐   │
│  │       MATRIX SELECTOR           │──┐── 122c
│  └────────────────────────────────┘   │
│  ┌────────────────────────────────┐   │
│  │        IMAGE FORMER             │──┐── 122d
│  └────────────────────────────────┘   │
└──────────────────────────────────────┘
```

## FIG. 7

START

CONVERT I/Q SIGNAL INTO FREQUENCY DOMAIN — 700

COMPUTE SKEWNESS FROM CONVERTED I/Q SIGNAL — 710

PERFORM CLUTTER FILTERING BASED ON SKEWNESS — 720

FORM COLOR FLOW IMAGE — 730

END

**FIG. 8**

710

721 — Is skewness equal to or greater than threshold value?

No →

722 — Perform clutter filtering of lowest cutoff

Yes ↓

723 — Perform clutter filtering of predetermined index

724 — Convert filtered I/Q signal into frequency domain

725 — Compute skewness

726 — Is skewness equal to or greater than threshold value?

Yes →

727 — Perform clutter filtering of subsequent index

No ↓

730

**FIG. 9**

START

PERFORM SINGULAR VALUE DECOMPOSITION WITH RESPECT TO I/Q SIGNAL — 900

GENERATE PLURALITY OF SUBMATRICES — 910

SELECT SUBMATRIX IN WHICH DOPPLER COMPONENT IS DOMINANT BASED ON SKEWNESS OF SUBMATRIX — 920

FORM COLOR FLOW IMAGE USING SELECTED SUBMATRICES — 930

END

# FIG. 10

910

CONVERT EACH OF SUBMATRICES INTO FREQUENCY DOMAIN — 921

COMPUTE SKEWNESS FROM EACH OF CONVERTED SUBMATRICES — 922

923

IS COMPUTED SKEWNESS EQUAL TO OR LESS THAN THRESHOLD VALUE?

NO

YES

SELECT CORRESPONDING SUBMATRIX — 924

925

ARE ALL SUBMATRICES PROCESSED?

NO

YES

930

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020100043154

- US 20040199078 A1

**Non-patent literature cited in the description**

- Reduction of the Clutter Component in Doppler Ultrasound Signals Based on Singular Value Decomposition: A Simulation Study. **LEDOUX L. A. F. et al.** ULTRASOUND IMAGING. Dynamedia Inc, 01 January 1997, vol. 19, 1-18